# EUROPÄISCHE PATENTANMELDUNG

(11) **EP 1 845 377 A1**
(43) Veröffentlichungstag der Anmeldung: **17.10.2007**
(21) Anmeldenummer: 06007478.8
(22) Anmeldetag: 10.04.2006
(51) Int. Cl.: G01N 33/558, G01N 33/543

(54) **Verfahren und Vorrichtung zum Bestimmen der Konzentrationen von Liganden**

(71) Anmelder: Micronas GmbH, 79108 Freiburg i. Br. (DE)
(72) Erfinder: Lehmann, Mirko, Dr. rer.nat.,, 79117 Freiburg (DE); Freund, Ingo, Dipl.-Ing. (FH), 79117 Freiburg (DE); Mohry, Sonja, 79108 Freiburg (DE); Klapproth, Holger, Dr., 79108 Freiburg (DE)
(74) Vertreter: Huwer, Andreas

(57) **Zusammenfassung**

Bel Verfahren zum Bestimmen der Konzentrationen von Liganden (9, 10) In einer zu analysierenden Probe sind Liganden (9) einer ersten Ligandenart für Rezeptoren (6) einer ersten Rezeptorart und Liganden (10) einer zweiten Ligandenart für Rezeptoren (8) einer zweiten Rezeptorart bindungsspezifisch. Ein erster Rezeptor (6) der ersten Rezeptorart wird an einer ersten Teststelle (5) und ein zweiter Rezeptor (8) der zweiten Rezeptorart an einer zweiten Teststelle (7) auf einem Substrat (23) immobllisiert. Ein dritter Rezeptor (11) der zweiten Rezeptorart wird derart mit der Probe vermischt, dass er in einer vorgegebenen Konzentration In dem so erhaltenen Gemisch vorliegt. Das Gemisch wird derart mit dem Substrat in Kontakt gebracht, dass mindestens ein Ligand (9) der ersten Ligandenart an den ersten Rezeptor (6) und Liganden (10) der zweiten Ligandenart an den zweiten Rezeptor (8) und den dritten Rezeptor (11) binden können. Danach werden Bestandteile des Gemischs, die nicht an einen immobilisierten Rezeptor (6, 8) gebunden sind, von dem Substrat entfernt Dann werden ein von der Konzentration des an den ersten Rezeptor (6) gebundenen Liganden (9) abhängiges erstes Messsignal und ein von der Konzentration des an den zweiten Rezeptor (8) gebundenen Liganden (10) abhängiges zweites Messsignal erzeugt. Mit Hilfe des ersten Messsignals wird die Konzentration des Liganden (9) der ersten Ligandenart und mit Hilfe des zweiten Messsignals und der Konzentration des dritten Rezeptors (11) in dem Gemisch die Konzentration der Liganden (10) der zweiten Ligandenart in der Probe bestimmt.

## Beschreibung

Die Erfindung betrifft ein Verfahren zum Bestimmen der Konzentrationen von Liganden in einer zu analysierenden Probe, wobei Liganden einer ersten Ligandenart für Rezeptoren einer ersten Rezeptorart und Liganden einer zweiten Ligandenart für Rezeptoren einer zweiten Rezeptorart bindungsspezifisch sind, wobei mindestens ein erster Rezeptor der ersten Rezeptorart an wenigstens einer ersten Teststelle und zumindest ein zweiter Rezeptor der zweiten Rezeptorart an wenigstens einer zweiten Teststelle auf einem Substrat immobilisiert werden. Ferner betrifft die Erfindung eine Vorrichtung zum Bestimmen der Konzentrationen von Liganden unterschiedlicher Ligandenarten in einer zu analysierenden Probe, mit einer zumindest eine Einlassöffnung und eine Auslassöffnung aufweisenden Messkammer, die ein Substrat hat, auf dem an einer ersten Teststelle ein erster Rezeptor einer ersten Rezeptorart und an einer zweiten Teststelle ein zweiter Rezeptor einer zweiten Rezeptorart immobilisiert sind, wobei die erste Rezeptorart für die erste Ligandenart für und die zweite Rezeptorart für die zweite Ligandenart bindungsspezifisch sind, wobei der ersten Teststelle ein erster Sensor zum Erfassen eines von der Konzentration des an den ersten Rezeptor gebundenen Liganden abhängigen ersten Messsignals und der zweiten Teststelle ein zweiter Sensor zum Erfassen eines von der Konzentration des an den zweiten Rezeptor gebundenen Liganden abhängigen zweiten Messsignals zugeordnet sind.

Eine derartige Vorrichtung ist aus US 6 197 503 B1 bekannt. Sie weist eine Durchfluss-Messkammer auf, die an ihrer Unterseite durch ein etwa plattenförmiges Substrat begrenzt ist, auf dem mehrere Teststellen angeordnet sind, an denen Rezeptoren immobilisiert sind. Die Rezeptoren sind jeweils für einen bestimmten, in einer zu analysierenden Probe enthaltenden Liganden bindungsspezifisch. An der Rückseite des Substrats ist ein Halbleiterchip vorgesehen, der an den einzelnen Teststellen jeweils einen optischen Sensor aufweist. Zur Anregung der Emission von Lumineszenzstrahlung in Abhängigkeit von der Bindung der Liganden an die Rezeptoren weist die Vorrichtung eine optische Strahlungsquelle auf, in deren Abstrahlbereich die Rezeptoren angeordnet sind. Zum Messen der Konzentration der Liganden wird die Probe durch eine Einlassöffnung hindurch derart in die Messkammer eingebracht, dass die in der Probe enthaltenen Liganden an die Rezeptoren binden können. Nach Ablauf einer vorbestimmten Einwirkungsdauer werden nicht an einen Rezeptor gebundene Bestandteile der Probe aus der Messkammer entfernt. Die Einwirkungsdauer wird so gewählt, dass nach dem Entfernen der ungebundnen Bestandteile nur ein Teil der an den einzelnen Teststellen jeweils vorhandenen Bindungsstellen eines Rezeptors an den für den betreffenden Rezeptor bindungsspezifischen Liganden gebunden ist. Dabei Ist das Verhältnis der gebundenen Bindungsstellen zu den freien Bindungsstellen von der Konzentration des betreffenden Liganden in der Probe abhängig.

In einem weiteren Verfahrensschritt wird eine Lösung in die Messkammer eingebracht, die Nachweisantikörper enthält, die mit einem Marker markiert sind. Die Nachweisantikörper sind für die Liganden bindungsspezifisch und binden an diese. Danach wird die Lösung aus der Messkammer entfernt, um die Teststellen mit Hilfe der Strahlungsquelle mit der Anregungsstrahlung zu bestrahlen. Die über die Liganden indirekt an die Rezeptoren gebundenen Nachweisantikörper werden durch die Anregungsstrahlung zur Aussendung einer Lumineszenzstrahlung angeregt, deren Wellenlänge sich von der Wellenlänge der Anregungsstrahlung unterscheidet. Mit Hilfe der optischen Sensoren, die für die Lumineszenzstrahlung empfindlich und für die Anregungsstrahlung unempfindlich sind, wird für jede Teststelle jeweils ein von der Lumineszenzstrahlung und somit der Konzentration der Liganden in der Probe abhängiges Messsignal erzeugt.

Die Vorrichtung hat den Nachteil, dass sie nur in einem begrenzten Konzentrationsbereich eine gleichzeitige Bestimmung der einzelnen Konzentrationswerte der unterschiedlichen, in der Probe enthaltenen Liganden ermöglicht. Bei bestimmten Proben, wie zum Beispiel Blutproben, kann es jedoch vorkommen, dass die in der Probe enthaltenen Liganden Konzentrationsunterschiede von bis zu sechs Dekaden aufweisen. Damit bei einer solchen Probe sowohl für den Liganden mit der geringsten Konzentration als auch für den Liganden mit der höchsten Konzentration jeweils ein Konzentrationswert bestimmt werden kann, ohne dass es an einer der Teststellen alle vorhandenen Rezeptor-Bindungsstellen an einen Liganden binden und somit der entsprechende Messwert in die Begrenzung gerät, werden mindestens zwei Versuche durchgeführt. Bei einem ersten, zur Bestimmung der Konzentration des den höchsten Konzentrationswert aufweisenden Liganden vorgesehenen Versuch wird eine geringe Einwirkungsdauer und bei einem zweiten, zur Bestimmung der Konzentration des den niedrigsten Konzentrationswert aufweisenden Liganden vorgesehenen Versuch, wird eine wesentlich größere Einwirkungsdauer gewählt als bei dem ersten Versuch. Nachteilig ist dabei, dass für jeden Versuch jeweils ein neuer Halbleiterchip benötigt wird, weshalb die Messung noch relativ teuer und arbeitsaufwändig ist. Ungünstig ist außerdem, dass eine entsprechend große Probenmenge benötigt wird. Bei der Untersuchung von Blutproben, die mit Hilfe eines Stechwerkzeugs aus dem Finger eines Patienten entnommen wird, kann dies bedeuten, dass an mindestens zwei Stellen Blut aus dem Finger entnommen werden muss, was besonders bei Kindern problematisch ist.

Es besteht deshalb die Aufgabe, eine Vorrichtung und ein Verfahren der eingangs genannten Art zu schaffen, die eine einfache, schnelle und kostengünstige Messung der Konzentration mehrerer Liganden ermöglichen, von denen vermutet wird, dass sie in einer zu untersuchenden Probe enthalten sind.

Diese Aufgabe wird bezüglich des Verfahrens dadurch gelöst, dass mindestens ein dritter Rezeptor der zweiten Rezeptorart derart mit der Probe vermischt wird, dass er in einer vorgegebenen Konzentration in dem so erhaltenen Gemisch vorliegt, dass das Gemisch derart mit dem Substrat in Kontakt gebracht wird, dass mindestens ein Ugand der ersten Ligandenart an den ersten Rezeptor und Liganden der zweiten Ligandenart an den zweiten Rezeptor und den dritten Rezeptor binden können, dass danach Bestandteile des Gemischs, die nicht an einen immobilisierten Rezeptor gebunden sind, von dem Substrat entfernt werden, dass dann ein von der Konzentration des an den ersten Rezeptor gebundenen Liganden abhängiges erstes Messsignal und ein von der Konzentration des an den zweiten Rezeptor gebundenen Liganden abhängiges zweites Messsignal erzeugt werden, und dass mit Hilfe des ersten Messsignals die Konzentration des Liganden der ersten Ligandenart und mit Hilfe des zweiten Messsignals und der Konzentration des dritten Rezeptors in dem Gemisch die Konzentration der Liganden der zweiten Ligandenart in der Probe bestimmt wird.

Der dritte Rezeptor bindet dabei den Liganden so, dass dieser nicht mehr an den zweiten Rezeptor binden kann. In vorteilhafter Welse ist es dadurch möglich, bei Proben, die mehrere, in ihrer Konzentration stark voneinander abweichende Liganden enthalten, die Konzentrationen der einzelnen Liganden mit nur einem einzigen Versuch zu bestimmen. Die Konzentration des dritten Rezeptors in dem aus der Probe und dem dritten Rezeptor bestehenden Gemisch und die Einwirkungsdauer, während der das Gemisch mit den Rezeptoren in Kontakt steht, werden dabei so auf die für die einzelnen Liganden zu überprüfenden Konzentrationsbereiche abgestimmt, dass nach dem Entfernen der nicht an einen Rezeptor gebundnen Bestandteile des Gemischs an allen Teststellen nur ein Teil der Bindungsstellen Rezeptoren an einen Liganden bindet, wenn dieser in dem zu untersuchenden Konzentrationsbereich in der Probe enthalten ist. Die Konzentration des zweiten Liganden kann mit Hilfe des Massenwirkungsgesetzes aus dem zweiten Messsignals und der bekannten Konzentration des dritten Rezeptors in dem Gemisch berechnet werden. Die Konzentration des dritten Rezeptors in dem Gemisch wird vorzugsweise so gewählt, dass sie einem Grenzwert der Konzentration des zweiten Liganden entspricht, aus dem sich eine Aussage, wie z.B. "gut/schlecht" oder "krank/gesund", ableiten lässt. Mit dem erfindungsgemäßen Verfahren können in einer Flusszelle oder dergleichen Messkammer nebeneinander Liganden-Konzentrationen in einem Bereich von g/l bis µg/l nachgewiesen werden.

Bei einer vorteilhaften Ausführungsform der Erfindung wird ein für den ersten Liganden bindungsspezifischer, mit einem ersten Marker markierter Nachweisantikörper mit der ersten Teststelle in Kontakt gebracht, wobei danach nicht an einen immobilisierten Rezeptor gebundene Marker von dem Substrat entfernt werden, und wobei danach das erste Messsignal in Abhängigkeit von der Konzentration des ersten Markers erzeugt wird. Die Konzentration des ersten Liganden kann also mit Hilfe eines Sandwich-Elisa gemessen werden.

Bei einer bevorzugten Ausgestaltung der Erfindung wird ein für die Liganden der zweiten Ligandenart bindungsspezifischer, mit einem zweiten Marker markierter Nachweisantikörper mit der zweiten Teststelle in Kontakt gebracht, wobei danach nicht an einen immobilisierten Rezeptor gebundene Marker von dem Substrat entfernt werden, und wobei dann das zweite Messsignal in Abhängigkeit von der Konzentration des zweiten Markers und der Konzentration des dritten Rezeptors In dem Gemisch erzeugt wird. Es kann also auch die Konzentration des ersten Liganden mit Hilfe eines Sandwich-Elisa gemessen werden.

Vorteilhaft ist, wenn der erste Marker und/oder zweite Marker ein Enzym ist (sind), wenn das Enzym während der Erfassung der Messsignale mit mindestens zwei Chemikalien in Kontakt gebracht wird, zwischen denen bei Anwesenheit des Enzyms eine chemische Redoxreaktion auftritt, und wenn das erste Messsignal und/oder das zweite Messsignal durch Messen eines Redoxpotentials erzeugt wird (werden). Dabei kann das Redoxpotential mit Hilfe eines ISFET gemessen werden.

Bei einer zweckmäßigen Ausgestaltung der Erfindung wird (werden) der erste Marker und/oder zweite Marker während der Erfassung der Messsignale mit einer Anregungsstrahlung bestrahlt, welche den (die) Marker zur Abgabe einer Lumineszenzstrahlung anregt, wobei das erste Messsignal und/oder das zweite Messsignal durch Messen der Lumineszenzstrahlung des betreffenden Markers erzeugt wird (werden). Dabei kann die Anregungsstrahlung mit Hilfe einer Lichtquelle, beispielsweise einer Leuchtdiode und/oder einer Xenon-Lampe erzeugt werden. Für den ersten und zweiten Marker wird vorzugsweise dieselbe Anregungsstrahlung verwendet. Es ist aber auch denkbar, dass der erste und zweite Marker unterschiedliche Farbstoffe, wie z.B. Cy3 und Cy5 sind, und dass diese Farbstoffe mit unterschiedlichen Weilenlängen angeregt werden.

Vorteilhaft Ist, wenn die erste Teststelle und/oder zweite Testelle während der Erfassung der Messsignale mit einem Chemilumineszenzsubstrat in Kontakt gebracht wird (werden), in dem in Abhängigkeit von der Bindung der Liganden der ersten Ligandenart an den ersten Rezeptor und/oder in Abhängigkeit von der Bindung der Liganden der zweiten Ligandenart an den zweiten Rezeptor eine Lumineszenzstrahlung angeregt wird, und wenn das erste Messsignal und/oder das zweite Messsignal durch Messen der Lumineszenzstrahlung an der betreffenden Teststelle erzeugt wird (werden). Die Lumineszenzstrahlung wird dabei ohne eine Anregungsstrahlung auf chemischem Wege erzeugt.

Die vorstehend genannte Aufgabe wird bezüglich der Vorrichtung dadurch gelöst, dass in der Messkammer mindestens ein nicht immobilisierter dritter Rezeptor der zweiten Rezeptorart derart angeordnet ist, dass er bei einem Kontakt mit der zu analysierenden Probe mit dieser ein Gemisch bildet, welches den dritten Rezeptor in vorgegebener Konzentration enthält, und dass der zweite Sensor mit einer Auswerteeinrichtung verbunden ist, die zum Bestimmen der Konzentration des zweiten Liganden in der Probe aus dem zweiten Messsignal und der vorgegebenen Konzentration des dritten Rezeptors in dem Gemisch ausgebildet ist.

Die Probe wird also während und/oder nach dem Eintritt In die Messkammer mit einem für den zweiten Liganden bindungsspezifischen Rezeptor der zweiten Rezeptorart vermischt, so dass der zweite Ligand durch den dritten Rezeptor weg gefangen und die verfügbare Konzentration des dieses Liganden dadurch reduziert wird. Wie bereits bei dem Verfahren erläutert wurde, können dadurch bei einer Probe, die mehrere, in ihrer Konzentration stark voneinander abweichende Liganden enthält, die Konzentrationen der einzelnen Liganden mit nur einem einzigen Versuch bestimmt werden.

Vorteilhaft ist, wenn der dritte Rezeptor in gelförmiger, teigiger oder fester Form in der Messkammer stabilisiert ist, vorzugsweise derart, dass er an einer Begrenzungswand der Messkammer anhaftet. Die Vorrichtung ist dadurch besonders gut handhabbar. Die Stabilisierung des Kompetitor umfasst vorzugsweise mindestens ein nlcht-reduzlerendes Disaccharid und mindestens ein Protein oder Polypeptid der LEA-Klasse. Das nicht-reduzierende Disaccharid kann aus einer Gruppe ausgewählt sein, die aus Trehaose (D-Glucopyranosyl-D-glucopyranose), Sucrose (B-D-Fructofuranosyl-a-D-glucopyranosid) sowie Derivaten davon ausgewählt sein kann. Eine derartige Stabilisierung ist in WO 2004/004455 A2 beschrieben. Der in stabilisierter Form vorliegende Kompetitor ist über längere Zeit haltbar.

Die vorstehend genannte Aufgabe wird bezüglich der Vorrichtung auch dadurch gelöst, dass die Vorrichtung eine Mischeinrichtung aufweist, die zum Vermischen der Probe mit mindestens einem dritten Rezeptor der zweiten Rezeptorart mit einer Zuführöffnung für die Probe und einem den dritten Rezeptor enthaltenden Aufnahmeraum verbunden ist, dass die Mischeinrichtung eine Abgabeöffnung für das aus der Probe und dem dritten Rezeptor gebildete Gemisch aufweist, die mit der Einlassöffnung der Messkammer verbunden ist, dass die Mischeinrichtung derart ausgestaltet ist, dass der dritte Rezeptor in einer vorgegebenen Konzentration in dem Gemisch vorliegt, und dass der zweite Sensor mit einer Auswerteeinrichtung verbunden ist, die zum Bestimmen der Konzentration des zweiten Liganden In der Probe aus dem zweiten Messsignal und der Konzentration des dritten Rezeptors in dem Gemisch ausgebildet ist.

Bei dieser Lösung wird die Probe also bereits vor dem Eintritt in die Messkammer mit Hilfe der Mischeinrichtung mit einem für den zweiten Liganden bindungsspezlfischen Rezeptor der zweiten Rezeptorart vermischt. Dadurch wird eine besonders homogene Verteilung des Rezeptors der zweiten Rezeptorart in der Probe ermöglicht. Die Konzentration des zweiten Liganden kann dadurch mit großer Präzision gemessen werden.

Vorteilhaft ist, wenn der dritte Rezeptor in gelförmiger, teigiger oder fester Form in dem Aufnahmeraum stabilisiert ist, vorzugsweise derart, dass er an einer Begrenzungswand des Aufnahmeraums anhaftet und dass der Aufnahmeraum zum Lösen des dritten Rezeptors in der Probe als Durchflussmischkammer ausgebildet ist, über welche die Zuführöffnung für die Probe mit der Einlassöffnung der Messkammer verbunden ist. Die Vorrichtung ist dadurch auf einfache Weise handhabbar.

Bei einer bevorzugten Ausgestaltung der Erfindung weist die Durchflussmischkammer eine Mischerstruktur auf, die derart ausgestaltet ist, dass das Gemisch beim Durchfluss durch die Durchflussmischkammer vorzugsweise In abwechselnd zueinander entgegengesetzt verlaufende Richtungen abgelenkt wird, wobei die Mischerstruktur zwischen dem in gelförmiger, teigiger oder fester Form vorliegen dritten Rezeptor und der Einlassöffnung der Messkammer angeordnet ist. Dabei kann der Mischer ein so genannter Möbiusmischer sein, der mit Methoden der Mikrosystemtechnik mit sehr kompakten Abmessungen hergestellt werden kann.

Bei einer zweckmäßigen Ausführungsform der Erfindung sind die Sensoren optische Sensoren, wobei der erste Rezeptor zur Detektion einer in Abhängigkeit von der Bindung der Liganden der ersten Ligandenart an den ersten Rezeptor erzeugten ersten Lumineszenzstrahlung vorzugsweise direkt auf dem ersten Sensor und/oder der zweite Rezeptor zur Detektion einer in Abhängigkeit von der Bindung der Liganden der zweiten Ligandenart an den zweiten Rezeptor erzeugten zweiten Lumineszenzstrahlung vorzugsweise direkt auf dem zweiten Sensor angeordnet ist (sind). Die an den Rezeptoren erzeugte Lumineszenzstrahlung kann dann direkt ohne den Umweg über eine Sammellinse in den (die) Sensor(en) eingekoppelt werden.

Die Vorrichtung kann Bestandteil eines Kits nach einem der Ansprüche 13 bis 18 sein, der zusätzlich zu der der Vorrichtung
- einen mit einem Enzym oder dergleichen Marker markierten, für den ersten und/oder zweiten Liganden bindungsspezifischen Nachweisantikörper,
- mindestens zwei Chemikalien, zwischen denen bei einem Kontakt mit dem an dem Nachweisantikörper angeordneten Enzym eine chemische Redoxreaktion auftritt, und/oder
- ein Chemilumineszenzsubstrat, in dem bei einem Kontakt mit dem an dem Nachweisantikörper angeordneten Enzym eine chemische Reaktion ausgelöst wird, bei der eine Lumineszenzstrahlung freigesetzt wird, und/oder
- eine Strahlungsquelle, die zur Aussendung der Lumineszenzstrahlung auf die erste Teststelle und/oder zweite angeordnet ist,
   aufweisen kann. Der Marker, das Chemilumineszenzsubstrat und/oder die Chemikalien können mit einer geeigneten Zuführeinrichtung, wie z.B. einer Mikropumpe oder einer Pipette, der Messkammer zugeführt werden.

Nachfolgend ist ein Ausführungsbeispiel der Erfindung anhand der Zeichnung näher erläutert. Es zeigen:
- Fig. 1: einen Längsschnitt durch ein erstes Ausführungsbeispiel einer Vorrichtung zum Bestimmen der Konzentrationen von In einer zu analysierenden Probe enthaltenen Liganden,
- Fig. 2: eine Teildarstellung einer Messkammer Vorrichtung, die mit einer zu analysierenden Probe befüllt ist, wobei auf einer Wandung der Messkammer immobilisierte Rezepotren und in der Probe enthaltene Liganden schematisch angedeutet sind,
- Fig. 3: eine Teildarstellung der Messkammer nach dem Markieren von Rezeptor-Ligandenkomplexen,

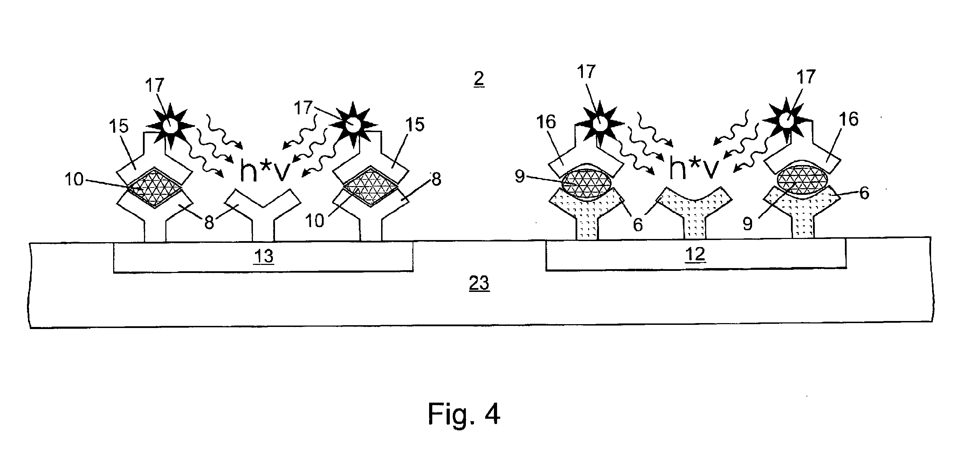

- Fig. 4: eine Teildarstellung der Messkammer nach dem Einbringen eines Chemi-Lumineszenzsubstrats in die Messkammer, und
- Fig. 5: einen Längsschnitt durch ein zweites Ausführungsbeispiel einer Vorrichtung zum Bestimmen der Konzentrationen von in einer zu analysierenden Probe enthaltenen Liganden.

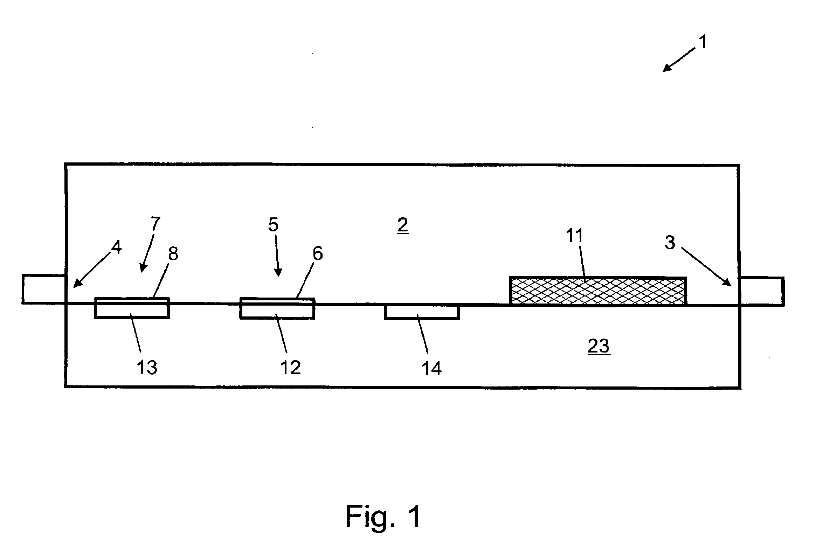

Eine In Fig. 1 im Ganzen mit bezeichnete Vorrichtung zum Bestimmen der Konzentrationen von mindestens zwei Liganden 9, 10 In einer zu analysierenden Probe weist eine als Flusszelle ausgebildete Messkammer 2 auf, die eine Einlassöffnung 3 und eine Auslassöffnung 4 hat. Eine Wandung der Messkammer 2 ist durch ein Halbleiter-Substrat 23 gebildet, auf dem an einer ersten Teststelle 5 mindestens ein erster Rezeptor 6 ersten Rezeptorart und an einer seitlich davon beabstandeten zweiten Teststelle 7 mindestens ein zweiter Rezeptor 8 einer zweiten Rezeptorart Immobilisiert sind. Der erste Rezeptor 6 ist für einen ersten, in der zu analysierenden Probe enthaltenen Liganden 9 und der zweite Rezeptor 8 für einen zweiten, in der Probe enthaltenen Liganden 10 bindungsspezifisch. Der zweite Ligand 10 weist eine größere Konzentration in der Probe auf als der erste Ligand 9.

In der Messkammer 2 ist ferner mindestens ein dritter Rezeptor 11 der zweiten Rezeptorart angeordnet, der nicht immobilisiert ist und in lyophiliserter Form vorliegt Der dritte Rezeptor 11 haftet an einer Begrenzungswand der Messkammer 2 an. Bei einem Kontakt mit der zu analysierenden wässrigen Probe löst sich der dritte Rezeptor 11 in der Probe.

An der ersten Teststelle 5 ist direkt unter dem ersten Rezeptor 6 ein erster optischer Sensor 12 und an der zweiten Teststelle 7 direkt unter dem zweiten Rezeptor 8 ein zweiter optischer Sensor 13 in das Halbleiter-Substrat 23 integriert. Zusätzlich ist ein dritter optischer Sensor 14 in der Wandung der Messkammer angeordnet, der nicht mit einem Rezeptor bedeckt ist und als Referenzwertgeber dient. Die Sensoren 12, 13, 14 können beispielsweise Photodioden sein.

Zum Bestimmen der Konzentrationen der Liganden 9, 10 wird zunächst die Probe durch die Einlassöffnung 3 hindurch derart In die Messkammer 2 eingefüllt, dass das gesamte Volumen der Messkammer 2 mit der Probe befüllt ist. Sobald der dritte Rezeptor 11 mit der Probe in Kontakt kommt, löst er sich in dieser. Der Lösungsvorgang kann ggf. durch Einringen von mechanischer Energie in die Messkammer 2 beschleunigt werden. Die Menge des dritten Rezeptors 11 ist derart auf das Volumen der Messkammer 2 abgestimmt, dass das aus der Probe und dem dritten Rezeptor 11 gebildete Gemisch den dritten Rezeptor 11 in einer vorgegebenen, einem zu messenden Grenzwert entsprechenden Konzentration enthält, wenn dieser vollständig und homogen in der Probe gelöst ist.

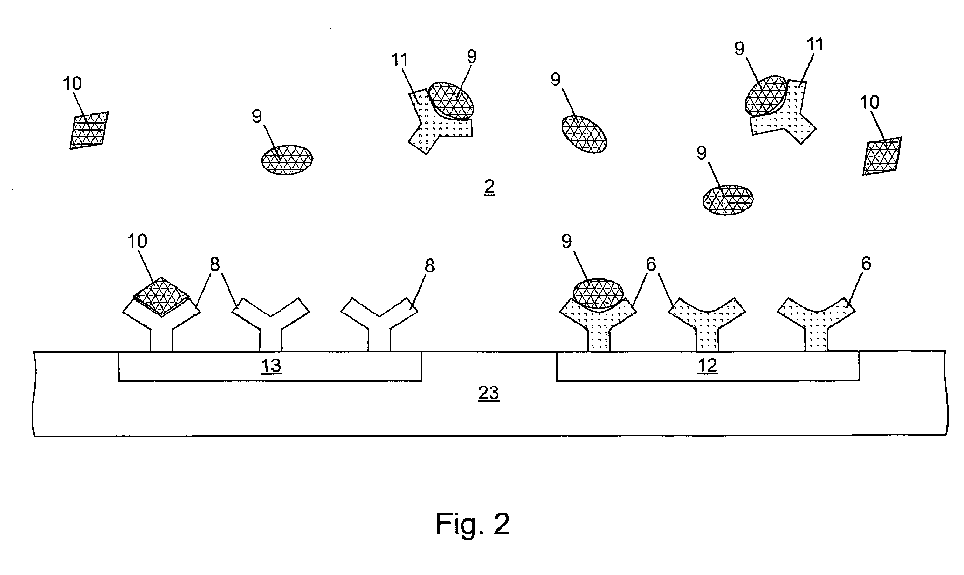

Nachdem die Messkammer 2 mit der Probe befüllt wurde, wird eine vorgegebene erste Zeitdauer abgewartet, während der sich der dritte Rezeptor 11 in der Probe lösen kann. Wie in Fig. 2 erkennbar ist, wird die erste Zeitdauer so gewählt, dass die in der Probe enthaltenen Liganden 9, 10 die Möglichkeit haben, jeweils an die für sie bindungsspezifischen Rezeptoren 6, 8, 10 zu binden. Deutlich ist erkennbar, dass jeweils nur ein Teil der an den einzelnen Teststellen 5, 7 vorhanden freien Bindungsstellen der Rezeptoren 6, 8 an einen Liganden 9, 10 bindet.

Nachdem die vorgegebene erste Zeitdauer abgelaufen ist, wird über die Zuführöffnung 14, die Einlassöffnung 3 und die Auslassöffnung 4 eine Spülflüssigkeit durch die Messkammer 2 hindurchgeleitet, welche die nicht an einen Rezeptor 6, 8 gebundenen Bestandteile des gebildeten Gemischs aus der Messkammer 2 entfernt.

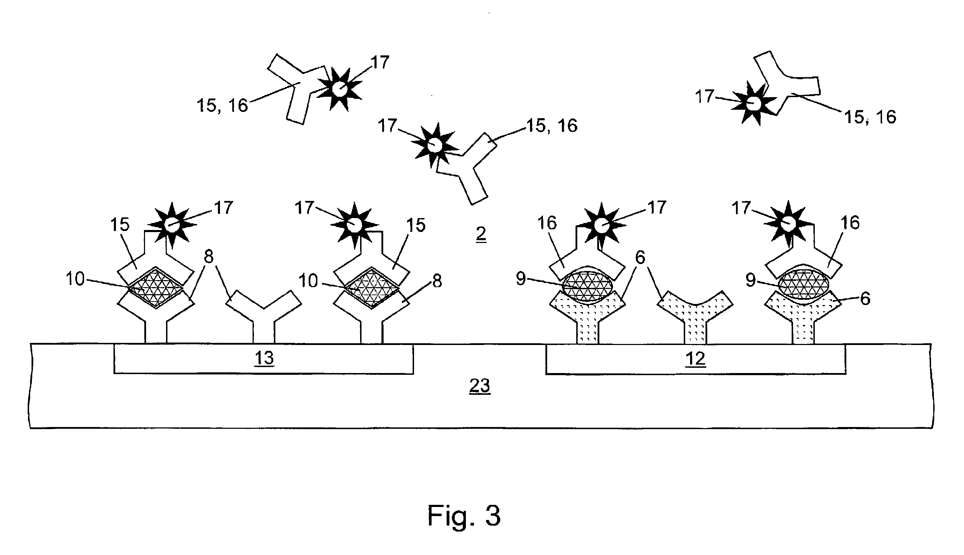

Danach wird eine Lösung in die Messkammer 2 eingebracht, die Nachweisantikörper 15, 16 enthält. Ein erster Nachweisantikörper 15 ist für Liganden 9 der ersten Ligandenart und ein zweiter Nachweisantikörper 15 ist für Liganden 10 der zweiten Ligandenart beindungsspezifisch. Die Nachweisantikörper 15, 16 sind jeweils mit einem Enzym 17, wie z.B. HRP, markiert Nachdem der Nachweisantikörper 15, 16 in die Messkammer 2 eingebracht wurde, wird eine vorgegebene zweite Zeitdauer abgewartet, die so gewählt ist, dass nahezu alle an den ersten Rezeptor 6 gebundenen ersten Liganden 9 jeweils an einen ersten Nachweisantikörpers 15 und nahezu alle an den zweiten Rezeptor 8 gebundenen zweiten Liganden 10 jeweils an einen zweiten Nachweisantikörper 15 binden und dadurch indirekt mit dem Enzym 17 markiert werden (Fig. 3).

Nachdem die vorgegebene zweite Zeitdauer abgelaufen ist, wird erneut Spülflüssigkeit durch die Messkammer 2 hindurchgeleitet, um nicht an einen Liganden 9, 10 gebundene Nachweisantikörper 15, 16 aus der Messkammer 2 zu entfernen.

Danach wird über die Einlassöffnung 3 ein Chemi-Lumineszenzsubstrat in die Messkammer 2 eingeleitet, das Wasserstoffperoxid und einen Chemilumlnophor, wie z.B. Luminol, enthält. Wenn das Enzym 17 mit dem Wasserstoffperoxid in Kontakt gerät, werden freie Sauerstoffradikale von dem Wasserstoffperoxid abgespalten, wodurch der Chemiluminophor unter Abgabe von Lumineszenzstrahlung chemisch zersetzt wird. Die Lumineszenzstrahlung wird also einerseits In Abhängigkeit von der Bindung des ersten Liganden 9 an den ersten Rezeptor 6 und andererseits in Abhängigkeit von der Bindung des zweiten Liganden 10 an den zweiten Rezeptor 8 in der Messkammer 2 erzeugt.

Die optischen Sensoren 12, 13 sind für die Lumineszenzstrahlung empfindlich und derart relativ zu den Rezeptoren 6, 8 angeordnet, dass sie jeweils nur die an der ihnen zugeordneten Teststelle 5, 7 erzeugte Lumineszenzstrahlung detektieren, nicht jedoch die Lumineszenzstrahlung, die an der jeweils anderen Teststelle 7, 5 erzeugt wird.

Mit den Sensoren 12, 13 ist eine in der Zeichnung nicht näher dargestellte Auswerteeinrichtung verbunden, die in Abhängigkeit von dem Messsignal des ersten Sensors 12 die Konzentrationen des ersten Liganden 6 In der Probe und In Abhängigkeit von dem Messsignal des zweiten Sensors 13 und der bekannten Konzentration des Kompetitors 15 in dem Gemisch mit Hilfe des Massenwirkungsgesetzes die Konzentration des zweiten Liganden 10 in der Probe bestimmt. Die Auswerteeinrichtung kann als elektrische Schaltung in das Substrat 23 bzw. die Wandung der Messkammer 2 Integriert sein.

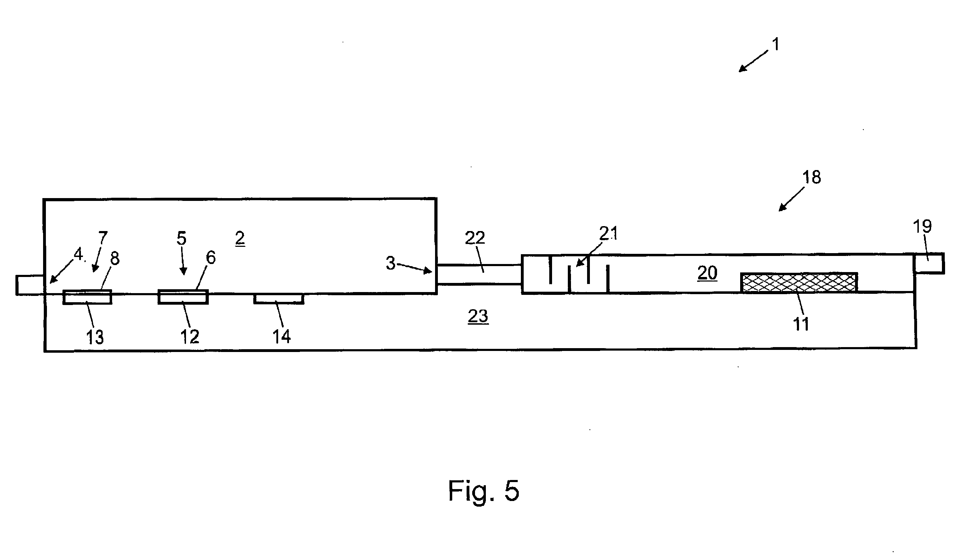

Bei dem in Fig. 5 gezeigten Ausführungsbeispiel weist die Vorrichtung 1 eine der Messkammer 2 vorgeschaltete Mischeinrichtung 18 auf, welche die Einlassöffnung 3 mit einer ZufiLihröffnung 19 verbindet. Zwischen der Zuführöffnung 19 und der Einlassöffnung 3 ist ein Aufnahmeraum 20 angeordnet, der den dritten Rezeptor 11 in stabilisierter Form enthält. Die Mischeinrichtung 13 dient dazu, die Probe mit dem dritten Rezeptor 11 homogen zu vermischen. Dazu wird die Probe beispielsweise mit Hilfe einer Pipette oder einer Pumpe durch die Zuführöffnung 19 hindurch in den Aufnahmeraum 20 eingeleitet. In dem Aufnahmeraum 20 ist der dritte Rezeptor 11 derart relativ zu der Zuführöffnung 19 angeordnet, dass die Probe während und/oder nach dem Einleiten in den Aufnahmeraum 19 mit dem dritten Rezeptor 11 in Kontakt gerät.

Die Mischeinrichtung 18 ist derart ausgestaltet, dass der dritte Rezeptor 11 in der Mischkammer 2 in einer vorgegebenen, einem zu messenden Grenzwert entsprechenden Konzentration in dem Gemisch vorliegt. Dies wird dadurch erreicht, dass die Messkammer 2 und die Mischeinrichtung 18 ein vorbestimmtes Volumen aufweisen, und dass die Menge des in dem Aufnahmeraum 20 stabilisierten dritten Rezeptors 11 so gewählt ist, dass sich die gewünschte Konzentration einstellt, wenn der dritte Rezeptor 11 mit der Probe zu einem Gemisch vermischt wird, welches das vorbestimmte Volumen aufweist.

In Strömungsrichtung hinter dem Aufnahmeraum 20 weist die Mischeinrichtung 18 eine als Möbiusmischer ausgestaltete Mischerstruktur 21 auf, die mit ihrem einen Ende mit dem Aufnahmeraum 20 und mit ihrem anderen, eine Abgabeöffnung für das aus der Probe und den dritten Rezeptoren 11 gebildete Gemisch aufweisenden Ende über einen Kanal 22 mit der Einlassöffnung 3 der Messkammer 2 verbunden ist.

Der Aufbau der Messkammer 2 entspricht im Wesentlichen dem in Fig. 1, jedoch mit dem Unterschied, dass in der Messkammer kein dritter Rezeptor 11 deponiert ist Insoweit wird auf die dortige Beschreibung verwiesen.

## Patentansprüche

1. Verfahren zum Bestimmen der Konzentrationen von Liganden (9, 10) in einer zu analysierenden Probe, wobei Liganden (9) einer ersten Ligandenart für Rezeptoren (6) einer ersten Rezeptorart und Liganden (10) einer zweiten Ligandenart für Rezeptoren (8) einer zweiten Rezeptorart bindungsspezifisch sind, wobei mindestens ein erster Rezeptor (6) der ersten Rezeptorart an wenigstens einer ersten Teststelle (5) und zumindest ein zweiter Rezeptor (8) der zweiten Rezeptorart an wenigstens einer zweiten Teststelle (7) auf einem Substrat (23) immobilisiert werden, wobei mindestens ein dritter Rezeptor (11) der zweiten Rezeptorart derart mit der Probe vermischt wird, dass er In einer vorgegebenen Konzentration In dem so erhaltenen Gemisch vorliegt, wobei das Gemisch derart mit dem Substrat (23) in Kontakt gebracht wird, dass mindestens ein Ligand (9) der ersten Ligandenart an den ersten Rezeptor (6) und Liganden (10) der zweiten Ligandenart an den zweiten Rezeptor (8) und den dritten Rezeptor (11) binden können, wobei danach Bestandteile des Gemischs, die nicht an einen immobilisierten Rezeptor (6, 8) gebunden sind, von dem Substrat (23) entfernt werden, wobei dann ein von der Konzentration des an den ersten Rezeptor (6) gebundenen Liganden (9) abhängiges erstes Messsignal und ein von der Konzentration des an den zweiten Rezeptor (8) gebundenen Liganden (10) abhängiges zweites Messsignal erzeugt werden, und wobei mit Hilfe des ersten Messsignals die Konzentration des Liganden (9) der ersten Ligandenart und mit Hilfe des zweiten Messsignals und der Konzentration des dritten Rezeptors (11) in dem Gemisch die Konzentration der Liganden (10) der zweiten Ligandenart in der Probe bestimmt wird.

2. Verfahren nach Anspruch 1, **dadurch gekennzeichnet, dass** ein für die Liganden (9) der ersten Ligandenart bindungsspezifischer, mit einem ersten Marker markierter Nachweisantikörper (15) mit der ersten Teststelle (5) in Kontakt gebracht wird, dass danach nicht an einen immobilisierten Rezeptor (6, 8) gebundene Marker von dem Substrat (23) entfernt werden, und dass dann das erste Messsignal In Abhängigkeit von der Konzentration des ersten Markers erzeugt wird.

3. Verfahren nach Anspruch 1 oder 2, **dadurch gekennzeichnet, dass** ein für die Liganden (10) der zweiten Ligandenart bindungsspezifischer, mit einem zweiten Marker markierter Nachweisantikörper (16) mit der zweiten Teststelle (7) in Kontakt gebracht wird, dass danach nicht an einen immobilisierten Rezeptor (11) gebundene Marker von dem Substrat (23) entfernt werden, und dass dann das zweite Messsignal in Abhängigkeit von der Konzentration des zweiten Markers und der Konzentration des dritten Rezeptors (11) in dem Gemisch erzeugt wird.

4. Verfahren nach einem der Ansprüche 1 bis 3, **dadurch gekennzeichnet, dass** der erste Marker und/oder zweite Marker ein Enzym ist (sind), dass das Enzym während der Erfassung der Messsignale mit mindestens zwei Chemikalien In Kontakt gebracht wird, zwischen denen bei Anwesenheit des Enzyms eine chemische Redoxreaktion auftritt, und dass das erste Messsignal und/oder das zweite Messsignal durch Messen eines Redoxpotentials erzeugt wird (werden).

5. Verfahren nach einem der Ansprüche 1 bis 4, **dadurch gekennzeichnet, dass** der erste Marker und/oder zweite Marker während der Erfassung der Messsignale mit einer Anregungsstrahlung bestrahlt wird (werden), welche den (die) Marker zur Abgabe einer Lumineszenzstrahlung anregt, und dass das erste Messsignal und/oder das zweite Messsignal durch Messen der Lumineszenzstrahlung des betreffenden Markers erzeugt wird (werden).

6. Verfahren nach einem der Ansprüche 1 bis 5, **dadurch gekennzeichnet, dass** die erste Teststelle (5) und/oder zweite Testelle (7) während der Erfassung der Messsignale mit einem Chemi-Lumineszenzsubstrat in Kontakt gebracht wird (werden), in dem in Abhängigkeit von der Bindung der Liganden (9) der ersten Ligandenart an den ersten Rezeptor (6) und/oder in Abhängigkeit von der Bindung der Liganden (10) der zweiten Ligandenart an den zweiten Rezeptor (8) eine Lumineszenzstrahlung angeregt wird, und dass das erste Messsignal und/oder das zweite Messsignal durch Messen der Lumineszenzstrahlung an der betreffenden Teststelle (5, 7) erzeugt wird (werden).

7. Vorrichtung (1) zum Bestimmen der Konzentrationen von Liganden (9, 10) unterschiedlicher Ligandenarten in einer zu analysierenden Probe, mit einer zumindest eine Einlassöffnung (3) und eine Auslassöffnung (4) aufweisenden Messkammer (2), die ein Substrat (23) hat, auf dem an einer ersten Teststelle (5) ein erster Rezeptor (6) einer ersten Rezeptorart und an einer zweiten Teststelle (7) ein zweiter Rezeptor (8) einer zweiten Rezeptorart immobilisiert sind, wobei die erste Rezeptorart für die erste Ligandenart für und die zweite Rezeptorart für die zweite Ligandenart bindungsspezifisch sind, wobei der ersten Teststelle (5) ein erster Sensor (12) zum Erfassen eines von der Konzentration des an den ersten Rezeptor (6) gebundenen Liganden (9) abhängigen ersten Messsignals und der zweiten Teststelle (7) ein zweiter Sensor (13) zum Erfassen eines von der Konzentration des an den zweiten Rezeptor (8) gebundenen Liganden (10) abhängigen zweiten Messsignals zugeordnet sind, **dadurch gekennzeichnet, dass** in der Messkammer (2) mindestens ein nicht immobilisierter dritter Rezeptor (11) der zweiten Rezeptorart derart angeordnet ist, dass er bei einem Kontakt mit der zu analysierenden Probe mit dieser ein Gemisch bildet, welches den dritten Rezeptor (11) in vorgegebener Konzentration enthält, und dass der zweite Sensor (13) mit einer Auswerteeinrichtung verbunden ist, die zum Bestimmen der Konzentration des zweiten Liganden (10) in der Probe aus dem zweiten Messsignal und der vorgegebenen Konzentration des dritten Rezeptors (11) in dem Gemisch ausgebildet ist.

8. Vorrichtung nach Anspruch 7, **dadurch gekennzeichnet, dass** der dritte Rezeptor (11) in gelförmiger, teigiger oder fester Form in der Messkammer (2) stabilisiert ist, vorzugsweise derart, dass er an einer Begrenzungswand der Messkammer (2) anhaftet.

9. Vorrichtung (1) nach dem Oberbegriff von Anspruch 7, **dadurch gekennzeichnet, dass** die Vorrichtung eine Mischeinrichtung (18) aufweist, die zum Vermischen der Probe mit mindestens einem dritten Rezeptor (11) der zweiten Rezeptorart mit einer Zuführöffnung (19) für die Probe und einem den dritten Rezeptor (11) enthaltenden Aufnahmeraum (20) verbunden ist, dass die Mischeinrichtung (18) eine Abgabeöffnung für das aus der Probe und dem dritten Rezeptor (11) gebildete Gemisch aufweist, die mit der Einlassöffnung (3) der Messkammer (2) verbunden ist, dass die Mischeinrichtung (18) derart ausgestaltet ist, dass der dritte Rezeptor (11) in einer vorgegebenen Konzentration in dem Gemisch vorliegt, und dass der zweite Sensor (13) mit einer Auswerteeinrichtung verbunden ist, die zum Bestimmen der Konzentration des zweiten Liganden (10) in der Probe aus dem zweiten Messsignal und der Konzentration des dritten Rezeptors (11) in dem Gemisch ausgebildet ist.

10. Vorrichtung (1) nach Anspruch 9, **dadurch gekennzeichnet, dass** der dritte Rezeptor (11) in gelförmiger, teigiger oder fester Form in dem Aufnahmeraum (20) stabilisiert ist, vorzugsweise derart, dass er an einer Begrenzungswand des Aufnahmeraums (20) anhaftet, und dass der Aufnahmeraum (20) zum Lösen des dritten Rezeptors (11) In der Probe als Durchflussmischkammer ausgebildet ist, über welche die Zuführöffnung (19) für die Probe mit der Einlassöffnung (3) der Messkammer (2) verbunden ist.

11. Vorrichtung (1) nach Anspruch 9 oder 10, **dadurch gekennzeichnet, dass** die Durchflussmischkammer eine Mischerstruktur (21) aufweist, die derart ausgestaltet ist, dass das Gemisch beim Durchfluss durch die Durchflussmischkammer vorzugsweise in abwechselnd zueinander entgegengesetzt verlaufende Richtungen abgelenkt wird, und dass die Mischerstruktur (21) zwischen dem in gelförmiger, teigiger oder fester Form vorliegenden dritten Rezeptor (11) und der Einlassöffnung (3) der Messkammer angeordnet ist.

12. Vorrichtung (1) nach einem der Ansprüche 7 bis 11, **dadurch gekennzeichnet, dass** die Sensoren (12, 13) optische Sensoren sind, und dass der erste Rezeptor (6) zur Detektion einer in Abhängigkeit von der Bindung der Liganden (9) der ersten Ligandenart an den ersten Rezeptor (6) erzeugten ersten Lumineszenzstrahlung vorzugsweise direkt auf dem ersten Sensor (12) und/oder der zweite Rezeptor (8) zur Detektion einer in Abhängigkeit von der Bindung der Liganden (10) der zweiten Ligandenart an den zweiten Rezeptor (8) erzeugten zweiten Lumineszenzstrahlung vorzugsweise direkt auf dem zweiten Sensor (13) angeordnet ist (sind).

13. Kit zur Durchführung des Verfahrens nach einem der Ansprüche 1 bis 6, umfassend
- eine Vorrichtung (1) nach einem der Ansprüche 7 bis 12, bei welcher der erste Sensor (12) ein Sensor zur Messung eines Redoxpotentials ausgebildet ist.
- einen für Liganden (9) der ersten Ligandenart bindungsspezifischen, mit einem Enzym markierten Nachweisantikörper (15) und
- mindestens zwei Chemikalien, zwischen denen bei einem Kontakt mit dem Enzym eine chemische Redoxreaktion auftritt.

14. Kit zur Durchführung des Verfahrens nach einem der Ansprüche 1 bis 6, umfassend
- eine Vorrichtung (1) nach einem der Ansprüche 7 bis 12, bei welcher der zweite Sensor (13) ein Sensor zur Messung eines Redoxpotentials ausgebildet ist,
- einen für Liganden (10) der zweiten Ligandenart bindungsspezifischen, mit einem Enzym markierten Nachweisantikörper (16) und
- mindestens zwei Chemikalien, zwischen denen bei einem Kontakt mit dem Enzym eine chemische Redoxreaktion auftritt.

15. Kit zur Durchführung des Verfahrens nach einem der Ansprüche 1 bis 6, insbesondere nach Anspruch 14, umfassend
- eine Vorrichtung (1) nach einem der Ansprüche 7 bis 12,
- einen für Liganden der ersten Ligandenart (9) bindungsspezifischen, mit einem Enzym markierten Nachweisantikörper und
- ein Chemi-Lumineszenzsubstrat, In dem bei einem Kontakt mit dem Enzym eine chemische Reaktion ausgelöst wird, bei der eine Lumineszenzstrahlung freigesetzt wird, für die der erste Sensor (12) empfindlich ist.

16. Kit zur Durchführung des Verfahrens nach einem der Ansprüche 1 bis 6, insbesondere nach Anspruch 13, umfassend
- eine Vorrichtung (1) nach einem der Ansprüche 7 bis 12,
- einen für Liganden (10) der zweiten Ligandenart bindungsspezifischen, mit einem Enzym markierten Nachweisantikörper (16) und
- ein Chemi-Lumineszenzsubstrat, in dem bei einem Kontakt mit dem Enzym eine chemische Reaktion ausgelöst wird, bei der eine Lumineszenzstrahlung freigesetzt wird, für die der zweite Sensor (13) empfindlich ist.

17. Kit zur Durchführung des Verfahrens nach einem der Ansprüche 1 bis 6, insbesondere nach Anspruch 14 oder 16, umfassend
- einen für Liganden der ersten Ligandenart bindungsspezifischen Nachweisantikörper (15), der mit einem Marker markiert ist, der durch Bestrahlen mit einer Anregungsstrahlung zur Abgabe einer Lumineszenzstrahlung anregbar ist,
- eine Vorrichtung (1) nach einem der Ansprüche 7 bis 10, bei welcher der erste Sensor (12) für die Lumineszenzstrahlung empfindlich und für die Anregungsstrahlung unempfindlich ist, und
- eine Strahlungsquelle, die zur Aussendung der Lumineszenzstrahlung auf die erste Teststelle (5) angeordnet ist.

18. Kit zur Durchführung des Verfahrens nach einem der Ansprüche 1 bis 6, insbesondere nach Anspruch 13 oder 15, umfassend
- einen für Liganden (9) der ersten Ligandenart bindungsspezifischen Nachweisantikörper (15), der mit einem Marker markiert ist, der durch Bestrahlen mit einer Anregungsstrahlung zur Abgabe einer Lumineszenzstrahlung anregbar ist,
- eine Vorrichtung (1) nach einem der Ansprüche 7 bis 10, bei welcher der zweite Sensor (13) für die Lumineszenzstrahlung empfindlich und für die Anregungsstrahlung unempfindlich ist, und
- eine Strahlungsquelle, die zur Aussendung der Lumineszenzstrahlung auf die zweite Teststelle (7) angeordnet ist.
